# EUROPÄISCHE PATENTSCHRIFT

(11) **EP 1 753 877 B1**
(45) Veröffentlichungstag und Bekanntmachung des Hinweises auf die Patenterteilung: **10.03.2010**
(21) Anmeldenummer: 05755065.9
(22) Anmeldetag: 27.05.2005
(51) Int. Cl.: C12Q 1/68, C12N 15/10

(54) **ERFINDUNG BETREFFEND cDNA-HERSTELLUNG AUS ZELLEN NACH LASER-MIKRODISSEKTION**
CDNA PRODUCTION FROM CELLS AFTER LASER MICRODISSECTION
PRODUCTION D'ADN COMPLEMENTAIRE A PARTIR DE CELLULES APRES MICRODISSECTION AU LASER

(30) Priorität: 28.05.2004 DE 102004026744
(43) Veröffentlichungstag der Anmeldung: 21.02.2007
(73) Patentinhaber: Philipps-Universität Marburg, 35032 Marburg (DE)
(72) Erfinder: LISS, Birgit, 35037 Marburg (DE)
(74) Vertreter: Meyer-Dulheuer, Karl-Hermann
(86) Internationale Anmeldenummer: PCT/DE2005/000979
(87) Internationale Veröffentlichungsnummer: WO 2005/116245

(56) Entgegenhaltungen:
- TIETJEN IAN ET AL: "Single-cell transcriptional analysis of neuronal progenitors." NEURON. 24 APR 2003, Bd. 38, Nr. 2, 24. April 2003 (2003-04-24), Seiten 161-175, XP002357852 ISSN: 0896-6273 in der Anmeldung erwähnt
- KAMME FREDRIK ET AL: "Single-cell microarray analysis in hippocampus CA1: demonstration and validation of cellular heterogeneity." THE JOURNAL OF NEUROSCIENCE : THE OFFICIAL JOURNAL OF THE SOCIETY FOR NEUROSCIENCE. 1 MAY 2003, Bd. 23, Nr. 9, 1. Mai 2003 (2003-05-01), Seiten 3607-3615, XP002357853 ISSN: 1529-2401 in der Anmeldung erwähnt
- BUSCHE S ET AL: "Expression of angiotensin AT(1) and AT(2) receptors in adult rat cardiomyocytes after myocardial infarction. A single-cell reverse transcriptase-polymerase chain reaction study." AMERICAN JOURNAL OF PATHOLOGY. AUG 2000, Bd. 157, Nr. 2, August 2000 (2000-08), Seiten 605-611, XP002357966 ISSN: 0002-9440
- HAHN SINUHE ET AL: "Single cell PCR in laser capture microscopy." METHODS IN ENZYMOLOGY. 2002, Bd. 356, 2002, Seiten 295-301, XP009058340 ISSN: 0076-6879
- TODD RANDY ET AL: "Challenges of single-cell diagnostics: analysis of gene expression." TRENDS IN MOLECULAR MEDICINE. JUN 2002, Bd. 8, Nr. 6, Juni 2002 (2002-06), Seiten 254-257, XP002357854 ISSN: 1471-4914

## Beschreibung

Die vorliegende Erfindung betrifft ein Verfahren zur kontaminationsfreien Isolierung einzelner Zellen aus fixiertem Gewebe durch Laser-Mikrodissektion und die anschließende analytische und präparative Untersuchung dieser Zellen, insbesondere die cDNA Synthese einzelner Neuronen aus fixierten Hirnschnitten mit Einzelzell-Sensitivität.

Beschreibung und Einleitung des allgemeinen Gebietes der Erfindung

Die Mikrodissektion ist ein Verfahren der Biowissenschaften, mit dem isolierte oder in einem Gewebe lokalisierte zelluläre, supra- und subzelluläre Strukturen abgetrennt werden und somit weiteren analytischen und präparativen Verfahren zugänglich gemacht werden können. Unter den Mikrodissektionsverfahren spielt das Laser Capture Microdissection-Verfahren kurz LCM genannt, eine besondere Rolle. Die Mikrodissektion wird manuell oder durch Mikromanipulation mechanisch in direktem Kontakt mit der Probe oder kontaktfrei mittels fokussierten Lasern durchgeführt. Die US 5998129 beschreibt ein Lasermikrodissektions-Verfahren, in dem aus einer auf einem planaren Objektträger aufgebrachten Gewebeprobe ein gewünschter Bereich z.B. Zellorganellen oder eine einzelne Zelle per Laserstrahl vom umgebenden Gewebe ausgeschnitten wird. Die sich noch auf dem Objektträger befindende isolierte Zelle wird mit einem zusätzlichen Laserimpuls in Richtung des Laserstrahl mobilisiert und in einem Reaktionsbehälter aufgefangen. Eines der weiteren analytischen und präparativen Verfahren mit Zellen, die durch Lasermikrodissektion isoliert wurden ist die Isolierung von Nukleinsäuren, DNA, RNA, insbesondere mRNA. Derzeit gibt es kommerzielle Kits beispielsweise der Firmen PALM (PALM RNA\Extraction Kit) und Qiagen (Qiagen RNAeasy Micro Kit), die die Isolierung von mRNA aus Lasermikrodissektierten Zellen ermöglicht. Allerdings liefern die mit diesen Kits durchgeführten keine akzeptablen reproduzierbaren Ergebnissen für isolierte einzelne Zellen.

Kamme Feta I (2003) J Neurosci 23(9) und Tietjen I et al (2003) Neuron 38 benutzen das Laser-Mikrodissektionsgerät der Firma Arcturus und beschreiben ein Verfahren, das zwar in der Anwendung und Ausbeute unkomplizierter als die kommerziellen Kits ist, aber keine kontaminationsfreie Sammlung einzelner spezifischer Zellen erlaubt. Insbesondere sind bei der Präparation von einzelnen Neuronen aus fixierten Hirnschnitten (z.B. postmortem human) Kontaminationen durch benachbarte Gliazellen problematisch. Derzeit ist kein Verfahren bekannt, mit welchem aus einzelnen Zellen nach spezifischer Lasermikrodissektion z.B. mit dem kontaktfreien PALM System mRNA in guter Qualität gewonnen werden kann.

Aufgabe der vorliegenden Erfindung ist es die beschriebenen Nachteile im Stand der Technik zu beheben und ein einfaches und schnelles Verfahren zur direkten cDNA-Synthese aus isolierten Zellen einer Gewebeprobe nach Lasermikrodissektion bereitzustellen.

Diese Aufgabe wird erfindungsgemäß gelöst durch ein Verfahren zur kombinierten Lyse und direkten cDNA-Synthese gemäß der Ansprüche. Das Verfahren beinhaltet folgende Schritte:
1 Bereitstellen von Zellen nach Laser-Mikrodissektion und Überführung in einen Reaktionsbehälter durch Katapultieren
2 Lyse der Zellen nach Schritt 1) und reverse Transkription der RNA aus den Zellen nach Schritt 1) in einem Lyse/cDNA-Synthese Reaktionspuffer, wobei beide Reaktionsschritte nacheinander in einem einzigen Reaktionsgefäß durchgeführt werden.

Das erfindungsgemäße Verfahren hat den Vorteil, dass es kostengünstig ist und schnell in wenigen Arbeitsschritten auch von weniger geschultem Laborpersonal durchgeführt wird. Entscheidend ist, dass der aufwendige und riskante Schritt der RNA Isolation entfällt und statt dessen im selben Reaktionsgefäß eine kombinierte Lyse und direkte cDNA-Synthese durchgeführt wird, die sichere kontaminationsfreie Ergebnisse liefert. Zur Detektion der cDNA ist eine direkte PCR im selben Reaktionsgefäß ohne weitere Aufreinigung oder andere zusätzliche Schritte möglich. Die Zusammensetzung des Puffers (aus NP40, Poly-I Carrier und Super RNAsin, sowie dNTPs und cDNA Syntheseprimer) als Lyse/cDNA-Synthese Reaktionspuffer ist besonders auf diese Anforderung abgestimmt. Weitere Schritte, wie Probenvorbereitung und Färbung, die Durchführung des Inkubationsprotokoll und Einhaltung der Temperaturvorgaben optimieren die RNA/cDNA Ausbeute.

Als Zellen werden intakte Zellen aus Suspension oder Kultur verwendet oder fixierte dehydrierte Schnittpräparate. Durch die Verwendung eines Trocknungsmittels z.B. Silica und den Zusatz eines Molekularsiebes in den Alkohol ist gewährleistet, dass die Gewebeprobe in sehr kurzer Zeit trocken genug für die Lasermikro-dissektion ist. Gleichzeitig minimiert die vollständige Dehydrierung Materialverluste durch RNAse Aktivität.

Vorteilhafterweise finden Lyse und cDNA-Synthese direkt im selben Reaktionsgefäß, sogar im selben Reaktionspuffer (Lyse/cDNA-Synthese Reaktionspuffer) statt. Eine gesonderte RNA Isolation ist nicht notwendig. Alternativ wird vor der cDNA-Synthese ein DNAse Verdau durchgeführt. Der erfindungsgemäße Lyse/cDNA-Synthese Reaktionspuffer beeinflusst eine anschliessende direkte qualitative PCR Amplifikation nicht, sodass auch diese im selben Reaktionsgefäß durchgeführt wird. Für die Durchführung einer quantitativen Real-time PCR Analyse wird alternativ ein Reinigungsschritt zwischengeschaltet, da die cDNA Reaktionskomponenten bekanntermaßen die Kinetik einer PCR Amplifikation stören. Derartige Reinigungsschritte sind dem Fachmann bekannt und können dem Stand der Technik z.B. aus Liss, Nuc Acids Research, 2002 entnommen werden.

### Ausführungsbeispiele

### 1. Bereitstellen von Zellen nach Laser-Mikrodissektion und Überführung in Reaktionsbehälter

Das-Bereitstellen einzelner fixierter Zellen oder einer einzelnen fixierten Zelle nach Laser-Mikrodissektion beinhaltet die Präparation einer geeigneten Gewebeprobe. Die Gewebeprobe stammt aus Organen und Geweben von Wirbeltieren, also Tieren und Menschen, die durch herkömmliche Verfahren wie Biopsie oder Präparation gewonnen werden, beispielsweise ist die Gewebeprobe eine Gehirnprobe eines Wirbeltieres, durch Präparation und Cryostat-Schneiden gewonnen. Dazu wird dem Wirbeltier z.B. einer Maus unter sterilen Bedingungen und auf Eis das Gehirn entnommen und Frontalcortex und caudales Kleinhirn entfernt. Das Mittelhirn wird auf einem Objekttisch mit Einbettmedium (z.B. tissue freezing medium der Firma Jung) versetzt und bei -20°C auf einer Schnellgefrierleiste (-45°C) eingefroren. Nach ca. 20 min werden bei einer Cryostattemperatur von -19°C Schnitte einer Schnittdicke von 12 µm gewonnen. Diese Schnitte werden anschließend auf einen sterilen Objektträger gebracht (z.B. PALM, PEN 1mm Glas).

Die Fixierung und Färbung der Gewebeprobe erfolgt z.B. durch Färbung der Schnitte mit Cresylviolett (z.B. Cresyl Violet acetate Staining Dye von Sigma, C 5042). Alternativ zu den gängigen Cresylviolett Färbeprotokollen wird eine sehr schnelle Färbung in einer 100 %igen alkoholischen Cresylviolettlösung bevorzugt. Daran schließt sich ein dem Fachmann bekanntes, jedoch verkürztes Verfahren mit Alkoholreihe an, wobei die Schnitte nach folgendem Schema inkubiert werden:

| | |
|---|---|
| 1. 75% Ethanol (-20°C vorgekühlt) | Dauer: 2 min |
| 2. Cresylviolett, | |
| einige Tropfen durch sterile Spritze mit Filter | Dauer: 30 sec |
| 3. 75% Ethanol | Dauer: 1-5 sec |
| 4. 100% Ethanol | Dauer: 1-5 sec |
| 5. 100% Ethanol auf einem Molekularsieb | Dauer: 1 min |

Alternativ werden auch andere, dem Fachmann bekannte Färbeverfahren durchgeführt. Vorteilhaft zur Erzielung einer möglichst vollständigen Dehydrierung der Gewebeprobe ist die Verwendung eines Molekularsiebes, beispielsweise das Molekularsieb der Firma VWR Merck Perlform 0,3nm und 2mm, Artikelnummer 1.057.041.000, im 100% Ethanol.

Nach der letzten Alkoholreihe werden die Schnitte in einer Box mit Trocknungsmittel z.B. Silica Gel getrocknet. Die Box mit Silica-Gel (Silica Gel with moisture indicator; Merck, 1 kg Best.No. 1.01925.1000) und das 100% Ethanol-Reaktionsgefäß wird zum längeren Lagern mit Parafilm abgedichtet.

Die Lasermikrodissektion einer einzelnen oder einzelner Zellen aus dem Gewebeschnitt wird nach Herstellerangaben z.B. PALM mit sterilen Geräten durchgeführt. Dazu werden beispielsweise der Laser und alle Geräte z.B. Pipetten, Zentrifuge, Vortex, Tisch zuerst mit RNase Zap, dann mit RNase freiem Wasser gereinigt. Es werden einzelne Zellen z.B. aus der Substantia nigra aus der vorbereiteten Gewebeprobe z.B. dem dehydrierten Gehirnschnitt ausgeschnitten und in Abhängigkeit vom Präparat z.B. bei einer Distanz von 2 mm und einer Katapult-Energie von 78% automatisch in einen Behälter oder Deckel (z.B. Adhesive Cap bei Geräten der Firma PALM) katapultiert. Letzterer hat den Vorteil, dass kein Puffer vorgelegt werden muss. Alternativ wird der sterile Lyse/cDNA-Synthese Reaktionspuffer aber auch in einem konventionellen Deckel zum Katapulting vorgelegt.

**2. Lyse der Zellen nach Schritt 1 und reverse Transkription der RNA aus den Zellen nach Schritt 1** in einem Lyse/cDNA-Synthese Reaktionspuffer und in einem Reaktionsgefäß

Die Zelle wird nach der Lasermikrodissektion im Behälter bzw. Deckel des Cap mit 4,5µl Lyse/cDNA-Synthese Reaktionspuffer versetzt, vorzugsweise wird dazu ein unter RNAse freien Bedingungen steril frisch angesetzter Puffer folgender Zusammensetzung verwendet:

| **Lyse/cDNA-Synthese Reaktionspuffer 1x** | **Menge** | **Endkonz.** |
|---|---|---|
| 5x first strand buffer (Invitrogen) | 1,00µl | 1x |
| 10x RT-Puffer (s.u.) | 0,50µl | 1x |
| DTT (Invitrogen; 0,1M) | 0,50µl | 10mM |
| Poly-I Carrier RNA (Sigma) 1µg/mg | 0,50µl | 500ng |
| Super-RNAsin (20 u/µl Ambion) | 0,50µl | 10u |
| Nonidet P40 (1:10 verdünnt; RNase-frei, Roche Diagnostics) | 0,25µl | 0,5% |
| H₂O | 1,25µl | |
| | | |
| **10x RT-Puffer** | **Konz.** | **Endkonz** |
| dNTPs (20mM each, Amersham Pharmacia Biotech) | 5mM | 0.5mM |
| random hexamer primer (1 mM, Roche Diagnostics) | 50µM | 5µM |
| Tris HCl pH 8, 100 mM | 10 mM | 1mM |

Als cDNA-Syntheseprimer werden beispielsweise random hexamer primer eingesetzt. Als RNA-Carrier wird beispielsweise Poly-I Carrier RNA verwendet. Dem Fachmann sind weitere Alternativen zu diesen Substanzen, die mit gleichem Effekt eingesetzt werden bekannt.

Auf den Deckel wird unter sterilen Bedingungen z.B. direkt ein PCR-Reaktionsgefäß aufgesetzt und 1,5 min bei 65°C im Wärmeschrank oder beheiztem geschlossenem System inkubiert, wobei das Reaktionsgefäß auf dem Deckel steht. Anschließend kommt die Probe im Deckel kurz auf Eis und wird kurz zentrifugiert, um dann erneut kurz auf Eis gestellt zu werden.
Nun wird Reverse Transcriptase beispielsweise Superscript (SuperScript II Reverse Transcriptase RNase H⁻ der Firma Invitrogen 100U/Reaktion) hinzugefügt und der Ansatz bei 37-38°C 2h inkubiert (Thermomixer Comfort Eppendorf; 38°C, Interval Mix). Vorzugsweise werden 0,5 µl Reverse Transcriptase eingesetzt.

Im Anschluss an die Lyse der Zellen und der reversen Transkription der RNA zu cDNA erfolgt die Detektion und Charakterisierung der cDNA durch Expressionsanalyse, dazu wird direkt eine qualitative (z.B. Multiplex) PCR durchgeführt. Alternativ wird der Ansatz auch sofort zur globalen PCR Amplifikation und anschliessenden Microarray Analyse eingesetzt. Für quantitative Genexpressionsanalyse wird ein Aufreinigungsschritt der Einzelzell cDNA (Liss, Nuc Acids Research, 2002) empfohlen.

Als Positivkontrolle dient z.B. hochverdünnte Midbrain-RNA oder Referenz-RNA (z.B. von Clontech oder Ambion), die genauso wie das Probenmaterial behandelt wird.

Die PCR dient der Amplifikation der gewünschten Gene aus den isolierten Einzelzellen beispielsweise für eine Nested PCR. Die Durchführung der PCR-Reaktion erfolgt unter Standardbedingungen, die Primer, Puffer und Inkubationszeiten sind dabei so ausgewählt, dass sie geeignet sind, die gewünschten Gene in guter Qualität zu amplifizieren.

Eine Ausführungsform der Erfindung betrifft einen Kit zur Lyse von Zellen aus einer Gewebeprobe nach Mikrodissektion und reverse Transkription der RNA, mindestens enthaltend
a) steril abgefüllten Lyse/cDNA-Synthese Reaktionspuffer
b) reverse Transcriptase

Auf Grund der Lehre der vorliegenden Erfindung sowie auf Grund des allgemeinen Fachwissens in diesem technischen Gebiet ist dem Hersteller des erfindungsgemäßen Kits bekannt, wie er die einzelnen Komponenten des Kits, z.B. die Puffer steril herstellt, formuliert und lagert.

Der Kit enthält, falls es für die Kundenfreundlichkeit gewünscht wird, auch weitere Materialien zur Dehydrierung der Gewebeprobe, wie 100% Alkohol mit Molekularsieb und Silicagel. Zusätzlich ist ein steriles Reaktionsgefäß mit geeignetem Dekkel zur Aufnahme der Zellen nach Mikrodissektion enthalten. Steril meint dabei, dass das Reaktionsgefäß RNase- und DNase frei ist.

## Patentansprüche

1. Verfahren zur kombinierten Lyse und cDNA Synthese aus RNA von einzelnen Zellen aus einer Suspension oder aus einer Kultur oder aus einem fixierten dehydrierten Schnittpräparat, **gekennzeichnet durch** die Schritte
1) Bereitstellen von Zellen nach Laser-Mikrodissektion und Überführung in einen Reaktionsbehälter **durch** Katapultieren
2) Lyse der Zellen nach Schritt 1) und reverse Transkription der RNA aus den Zellen nach Schritt 1) in einem Lyse/cDNA-Synthese Reaktionspuffer,
wobei beide Reaktionsschritte nacheinander in einem einzigen Reaktionsgefäß durchgeführt werden.

2. Verfahren gemäß Anspruch 1, **dadurch gekennzeichnet, dass** Lyse und reverse Transkription in einem sterilen Reaktionsgefäß mit Deckel durchgeführt werden.

3. Verfahren gemäß Anspruch 1, **dadurch gekennzeichnet, dass** Lyse und reverse Transkription in einem sterilen Lyse/cDNA-Synthese Reaktionspuffer enthaltend NP40, Carrier RNA Poly-I, Super RNAsin, sowie dNTPs und cDNA Syntheseprimer durchgeführt werden.

4. Verfahren gemäß einem der vorangegangenen Ansprüche, **dadurch gekennzeichnet, dass** zwischen Lyse und cDNA-Herstellung eine DNAse Reaktion stattfindet.

5. Verfahren gemäß einem der vorangegangenen Ansprüche, **dadurch gekennzeichnet, dass** die einzelnen Zellen einer biologischen Probe entstammen.

6. Verfahren gemäß einem der vorangegangenen Ansprüche, **dadurch gekennzeichnet, dass** die biologische Probe Gewebe oder Organ eines Wirbeltieres ist.

7. Verfahren gemäß einem der vorangegangenen Ansprüche, **dadurch gekennzeichnet, dass** die biologische Probe mit einem zur Gewebefärbung geeigneten Farbstoff, insbesondere mit einer alkoholischen Cresylviolettlösung gefärbt ist.

8. Verfahren gemäß einem der vorangegangenen Ansprüche, **dadurch gekennzeichnet, dass** die biologische Probe ein dehydrierter Gewebeschnitt ist.

9. Verfahren gemäß einem der vorangegangenen Ansprüche, **dadurch gekennzeichnet, dass** die Dehydrierung in 100% Ethanol mit einem Molekularsieb erfolgt.

10. Verfahren gemäß einem der vorangegangenen Ansprüche, **dadurch gekennzeichnet, dass** die dehydrierte biologische Probe mit einem Trocknungsmittel behandelt wird.

11. Verfahren gemäß Anspruch 10, **dadurch gekennzeichnet, dass** das Trocknungsmittel Silica-Gel ist.

12. Kit zur Lyse von Zellen aus einer Suspension oder aus einer Kultur oder aus einem fixierten, dehydrierten Schnittpräparat nach Gewinnung der Zellen durch Mikrodissektion und zur reversen Transkription das Kit mindestens enthaltend
- Lyse/cDNA-Synthese Reaktionspuffer und
- reverse Transkriptase,
wobei der Lyse/cDNA-Synthese Reaktionspuffer steril ist und für eine Reaktion
- 10 mM DTT,
- 500 ng Poly-I Carrier RNA,
- 10 U Super-RNAsin,
- 0,5 % Nonidet P40,
- 0,5 mM dNTPs und
- 5 µM random hexamer Primer
- **1x First strand buffer**
- **1mM Tris HCl, pH8**
enthält.

13. Kit nach Anspruch 12, **dadurch gekennzeichnet, dass** er zusätzlich noch DNAse enthält.

14. Kit gemäß den Ansprüchen 12 bis 13, **dadurch gekennzeichnet, dass** er eine 100% Alkohol-Lösung mit Molekularsieb und Silicagel enthält.

15. Kit gemäß den Ansprüchen 12 bis 14, **dadurch gekennzeichnet, dass** er ein steriles Reaktionsgefäß mit Deckel enthält.

## Claims

1. A method for combined lysis and cDNA synthesis of RNA from single cells in a suspension or cells of a tissue sample or cells of a fixed and dehydrated sample slice, **characterized by** the steps of
1) Preparing cells after laser microdissection and transferring the cells into a reaction tube through catapulting;
2) Lysing cells in step 1) and reverse transcribing the RNA from the cells in step 1) in a lysis/cDNA-synthesis reaction buffer,
wherein these two steps are carried out one after the other in a single reaction tube.

2. The method according to claim 1, **characterized in that** the lysis and reverse transcription steps are carried out in a sterile reaction tube with a lid.

3. The method according to claim 1, **characterized in that** the lysis and reverse transcription steps are carried out in a sterile lysis/cDNA-synthesis reaction buffer comprising NP40, carrier RNA, Super RNAsin as well as dNTPs and cDNA synthesis primer.

4. The method according to claim 1, **characterized in that** between the lysis and cDNA-synthesis steps, a DNAse reaction is carried out.

5. The method according to claim 1, **characterized in that** the single cells derive from a biological specimen.

6. The method according to claim 5, **characterized in that** the biological specimen is a tissue or an organ of a vertebrate.

7. The method according to claim 5, **characterized in that** the biological specimen is stained with a dye suitable for tissue staining, especially with cresyl violet dissolved in an alcohol.

8. The method according to claim 5, **characterized in that** the biological specimen is a dehydrated tissue section.

9. The method according to claim 8, **characterized in that** the dehydration is carried out in 100% ethanol with a molecular sieve.

10. The method according to claim 8, **characterized in that** the dehydrated biological specimen are treated with a drying agent.

11. The method according to claim 10, **characterized in that** the drying agent is silica gel.

12. A kit for lysing cells in a suspension or cells of a tissue sample or cells of a fixed and dehydrated sample slice after microdissection of a biological tissue specimen and for reverse transcription, comprising at least
- Lysis/cDNA-synthesis reaction buffer and
- reverse transcriptase,
wherein the lysis/cDNA-synthesis reaction buffer is sterile and comprises
- 10 mM DTT,
- 500 ng Poly-I Carier RNA,
- 10 U Super-RNAsin,
- 0.5% Nonidet P40,
- 0.5 mM dNTPs and
- 5 µM random hexamer Primer
- 1 x First strand buffer
- 1mM Tris HCl, pH 8
for one reaction.

13. The kit according to claim 12, **characterized in that** it contains additionally DNAse.

14. The kit according to claims 12 and 13, **characterized in that** it contains further a 100% ethanol solution with molecular sieve and silica gel.

15. The kit according to claims 12 to 14, **characterized in that** it contains a sterile reaction tube with lid.

## Revendications

1. Procédé permettant de réaliser de façon combinée la lyse et la synthèse d'ADNc à partir d'ARN provenant de cellules isolées d'une suspension ou d'une culture ou d'une coupe déshydratée et fixée, **caractérisé par** les étapes suivantes
1) mise à disposition de cellules suite à la micro-dissection laser et transfert dans un réacteur par catapultage
2) lyse des cellules obtenues à l'étape 1) et transcription inverse de l'ARN provenant des cellules obtenues à l'étape 1) dans un tampon de réaction de lyse / de synthèse d'ADNc,
les deux étapes de réaction étant réalisées successivement dans un même réacteur.

2. Procédé selon la revendication 1, **caractérisé en ce que** la lyse et la transcription inverse sont réalisées dans un réacteur stérile muni d'un couvercle.

3. Procédé selon la revendication 1, **caractérisé en ce que** la lyse et la transcription inverse sont réalisées dans un tampon de réaction de lyse / de synthèse d'ADNc contenant du NP40, du Carrier RNA Poly-1, du Super RNAsin ainsi que des dNTP et des amorces de synthèse d'ADNc.

4. Procédé selon l'une des revendications précédentes, **caractérisé en ce qu'**une réaction de DNAse a lieu entre la lyse et la production d'ADNc.

5. Procédé selon l'une des revendications précédentes, **caractérisé en ce que** lesdites cellules isolées proviennent d'un échantillon biologique.

6. Procédé selon l'une des revendications précédentes, **caractérisé en ce que** ledit échantillon biologique est un tissu ou un organe d'un vertébré.

7. Procédé selon l'une des revendications précédentes, **caractérisé en ce que** ledit échantillon biologique est coloré avec un colorant approprié pour la coloration de tissus, notamment avec une solution alcoolique de violet de crésyl.

8. Procédé selon l'une des revendications précédentes, **caractérisé en ce que** ledit échantillon biologique est une coupe de tissu déshydratée.

9. Procédé selon l'une des revendications précédentes, **caractérisé en ce que** la déshydratation est réalisée dans de l'alcool à 100% avec un tamis moléculaire.

10. Procédé selon l'une des revendications précédentes, **caractérisé en ce que** ledit échantillon biologique déshydraté est traité avec un agent de déshydratation.

11. Procédé selon la revendication 10, **caractérisé en ce que** ledit agent de déshydratation du gel de silice.

12. Kit destiné à la lyse de cellules provenant d'une suspension ou d'une culture ou d'une coupe déshydratée et fixée, lesdites cellules ayant été prélevées par micro-dissection, et à la transcription inverse, ledit kit contenant au moins
- du tampon de réaction de lyse / de synthèse d'ADNc et
- de la transcriptase inverse,
ledit tampon de réaction de lyse / de synthèse d'ADNc étant stérile et contenant pour une réaction
- 10 mM de DTT
- 500 ng de Poly-I Carrier RNA
- 10 U de Super-RNAsin,
- 0,5 % de Nonidet P40,
- 0,5 mM de plusieurs dNTP et
- 5 µM d'une amorce de type random hexamer
- 1 x tampon de type first strand
- 1mM Tris HCl, pH 8

13. Kit selon la revendication 12, **caractérisé en ce qu'**il contient de façon supplémentaire de la DNAse.

14. Kit selon les revendications 12 à 13, **caractérisé en ce qu'**il contient une solution alcoolique à 100 % avec tamis moléculaire et gel de silice.

15. Kit selon les revendications 12 à 14, **caractérisé en ce qu'**il contient un réacteur stérile muni d'un couvercle.
